# EUROPEAN PATENT APPLICATION

(11) **EP 2 098 174 A2**
(43) Date of publication of application: **09.09.2009**
(21) Application number: 09154473.4
(22) Date of filing: 05.03.2009
(51) Int. Cl.: A61B 17/12, A61L 31/00

(54) **Multiple biocompatible polymeric strand aneurysm embolization system and method**

(30) Priority: 05.03.2008 US 42515
(71) Applicant: Neurovasx, Inc., Maple Grove, MN 55369 (US)
(72) Inventor: Calabria, Marie, Plymouth Minnesota 55446 (US); Bertelson, Arthur J., Buffalo Minnesota 55313 (US)
(74) Representative: Curley, Donnacha John

(57) **Abstract**

Some embodiments includes a system that includes a catheter including a distal portion for location at least partially in an aneurysm, the catheter including a proximal portion, the catheter to transport a biocompatible polymeric strand to an aneurysm, a biocompatible polymeric strand sized to slide through the catheter and a heated excise collar coupled to the distal portion of the catheter, the heated excise collar to provide radial heat from the catheter inward to melt and excise the biocompatible polymeric strand.

## Description

### BACKGROUND

An aneurysm is a balloon-like swelling in a wall of a blood vessel. Aneurysms result in weakness of the vessel wall in which they occur. This weakness predisposes the vessel to tear and possibly rupture which can harm the individual suffering from the aneurysm. Vascular aneurysms are a result of an abnormal dilation of a blood vessel that can result from disease, genetic predisposition and other afflictions which weaken the arterial wall, allowing it to expand.

Aneurysms in cerebral circulation can occur in an anterior communicating artery, posterior communicating artery, and a middle cerebral artery. Aneurysms can occur at the curvature of these vessels or at bifurcations of these vessels, among other places. The majority of cerebral aneurysms occur in women. Cerebral aneurysms are often diagnosed by the rupture and subarachnoid bleeding of the aneurysm.

### DESCRIPTION OF THE DRAWINGS

FIG. 1 is cross-section side view of a catheter deploying a biocompatible polymeric weave embolization device, according to an embodiment of the present subject matter

FIG. 2 illustrates a biocompatible polymeric weave embolization device including biocompatible polymeric strands joined at an end, according to an embodiment of the present subject matter.

FIG. 3 illustrates an embodiment of a biocompatible polymeric weave embolization device including biocompatible polymeric strands joined together and joined at an end.

FIG. 4 illustrates an embodiment of a biocompatible polymeric weave embolization device in a weave, according to an embodiment.

FIG. 5 illustrates a cross-section of two biocompatible polymeric strands of different diameters, according to an embodiment.

FIG. 6 illustrates a cross-section of three biocompatible polymeric strands of different diameters, according to an embodiment.

FIG. 7 illustrates a cross-section of four biocompatible polymeric strands of different diameters, according to an embodiment.

FIG. 8 illustrates a cross-section of five biocompatible polymeric strands of different diameters, according to an embodiment.

FIG. 9 illustrates a biocompatible polymeric strand including an irregular lumen, according to an embodiment.

FIG. 10 illustrates a solid biocompatible polymeric strand having an irregular shape, according to an embodiment.

FIG. 11 illustrates a pusher for pushing through a catheter, according to an embodiment.

FIG. 12 illustrates a biocompatible polymeric weave and a pusher, according to an embodiment.

FIG. 13 illustrates an expanded weave embolization device in use, according to an embodiment.

FIG. 14 illustrates a catheter including an heated excise collar with a biocompatible polymeric strand slidably disposed in the catheter, according to an embodiment.

FIG. 15 illustrates a weave and a conductive ring, according to an embodiment.

FIG. 16 illustrates a catheter having a conductive ring, according to an embodiment.

FIG. 17 illustrates a catheter having a heated excise collar demonstrating isotropic heating, according to an embodiment.

FIG. 18 illustrates a method of embolization, according to an embodiment.

### DETAILED DESCRIPTION

The present systems and methods provide, in various embodiments, a system that includes a catheter for location at least partially in an aneurysm. In various embodiments the catheter transports a biocompatible polymeric strand or a weave of biocompatible polymeric strands to an aneurysm to fill the aneurysm. In some examples, a heated excise collar is coupled to the catheter. In some examples, this collar provides radial heat from the catheter inward to melt and excise the biocompatible polymeric strand or the weave.

Current treatment options for cerebral aneurysm fall into at least two categories: surgical and interventional. The surgical option has been a standard of care for the treatment of aneurysms. Surgical treatment involves high risk and a long period of postoperative rehabilitation and critical care. Successful surgery allows for an endothelial-cell to endothelial-cell closure of the aneurysm and therefore a cure for the disease. If an aneurysm is present within an artery in the brain and bursts, this creates a subarachnoid hemorrhage, possibility resulting in death. Even with successful surgery, recovery takes several weeks and often requires a long hospital stay.

In order to overcome some of these drawbacks, interventional methods and prostheses have been developed to provide an artificial structural support to the vessel region impacted by the aneurysm. The structural support should have an ability to maintain its integrity under blood pressure conditions and impact pressure within an aneurismal sac and thus prevent or minimize a chance of rupture.

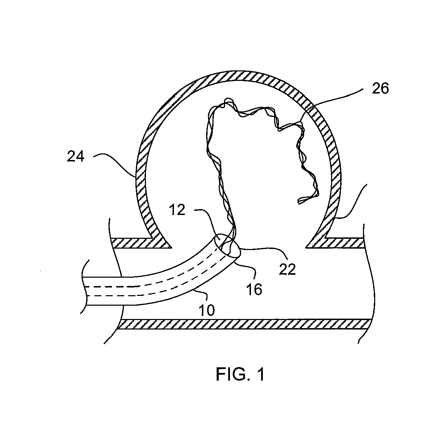

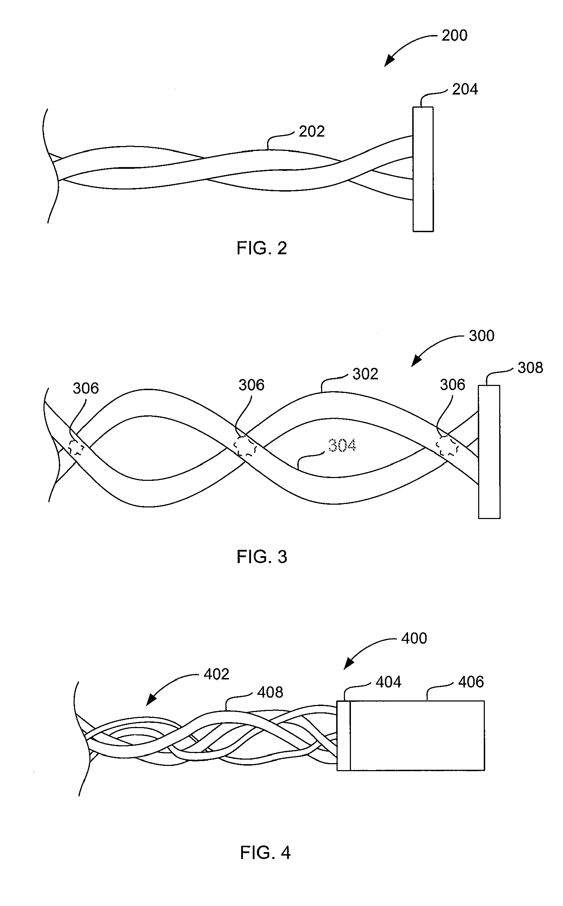

FIG. 1 illustrates a cross-section side view of some embodiments of a catheter used for repairing an aneurysm, according to some examples. The catheter is part of a system to transport a biocompatible polymeric strand or strands 26, such as is shown schematically in FIG. 2. Biocompatible polymeric strand or strands 26, in some examples, include materials that are positionable within an aneurysm 24 to fill or plug the aneurysm 24. These biocompatible polymeric strand or strands 26 seal the aneurysm 24 from an opening in vasculature, in some examples.

The catheter includes a distal portion 16 for location at least partially in the aneurysm, and further includes a proximal portion. The proximal portion is not implanted during surgery, in some examples. The biocompatible polymeric strand or strands 26 are deployed to an aneurysm 24 through a lumen, illustrated at 12 which opens to the aneurysm 24. The lumen 12 is defined by a catheter, such as is illustrated at 10 in FIG. 1. The proximal portion comprises a manifold with a port for insertion of the biocompatible polymeric strand or strands 26.

FIG. 1 is cross-section side view of a catheter deploying biocompatible polymeric strands in a weave, according to an embodiment of the present subject matter. One or more biocompatible polymeric strand 26 are pushed through the lumen 12 to the distal portion 16. The distal portion 16, in some embodiments, terminates in a curved tip 22. The curved tip 22 is positionable within an aneurysm 24. A straight tip is used in some embodiments. Other tip shapes are used in additional examples.

The lumen 12 of catheter 10 has a generally circular cross-sectional configuration with an external diameter in a range of about 0.01 to 0.05 inches for cerebral vascular applications. The lumen 12 has sufficient structural integrity to permit the catheter 10 to be advanced to distal arterial locations without buckling or undesirable bending of the lumen 12. In some examples, the lumen 12 of catheter 10 has a generally circular cross-sectional configuration with an internal diameter ranging from 0.01 to 0.035 inches.

As used herein, the terms "biocompatible polymeric strand or strands," "biocompatible weave," and "biocompatible polymeric sleeve" are used to describe types of aneurysm detachable filler. The biocompatible polymeric weave illustrated is formed to make a long continuous string-shape. The string-shape is placed into an aneurysm in a continuous fashion until angiographic filling is achieved. The string-shaped filler is then excised. Optionally, in embodiments that include a strand, the strand can be coiled to define an elongate tube. The coil can include individual coils which abut, in some examples.

In some examples, the biocompatible polymeric strand includes material selected from one or more of an acrylamide, a methacrylate, cyclodextran, synthetic elastin polymer, poly chelating amphiphilic polymer, hydrogel, hyaluronic acid conjugate, natural hydrogel, a synthetic hydrogel, silicone, polyurethane, polysulfone, cellulose, polyethylene, polypropylene, polyamide, polyimide, polytetrafluoroethylene, polyvinyl chloride, epoxy, phenolic, neoprene, polyisoprene, one or more of a thermoresponsive polymer, a pH sensitive polymer, or a shape memory polymer and a combination thereof.

Thermoresponsive polymers are polymers that swell upon reaching body temperature but do not swell by hydration. pH sensitive polymers swell upon reaching physiologic pH values. Shape memory polymers are polymers which are given a shape outside the body. Shape memory polymers return to an original shape with either hydration, thermal or pH changes. Each of these types of swellable polymers, those swellable by hydration, those swellable by heat and those swellable by pH are suitable for use in embodiments of the methods and apparatuses described herein.

Hydrogel materials suitable for use in the aneurysm biocompatible polymeric strand methods and apparatuses described herein include n-vinyl pyrrolidone, acrylic acid, sodium acrylate, acrylamido methyl propanesulfonic acid, sulfopropyl acrylate potassium salts, acryloyoxy ethyltrimethyl-ammonium methyl sulfate, albumin and gelatin modified by sulfate and poly (met acrylic acid) poly isopropyl acrylamide. Biocompatible polymeric strand materials also include hyaluronic acid conjugates, polyanhydrides, glycolipids, polysaccharides, and halamines, silicone, polysulfone, polyamide, polyimide, polyester, polytetrafluoroethylene, polyvinyl chloride, epoxy, phenolic, neoprene, polyisoprene and a combination thereof. For some embodiments, the biocompatible polymeric strand includes a polymer that has a melt temperature of between 200 to 600°F. For some embodiments, the biocompatible polymeric strand includes a conductive polymer that has a melt temperature of 200 to 600°F.

Biocompatible polymeric strands include biodegradable materials such as polylactic acid ("PLA"), poly (y-glutamic acid) ("PGA"), polyanhydrides and other similar biodegradable materials, in various embodiments. Some embodiments include one or more bioactive compounds selected from a group that includes an antithrombotic agent, an antiplatelet agent, an antimitotic agent, an antioxidant, an antimetabolite agent, an antiinflammatory agent, and a combination thereof.

In some embodiments, the biocompatible polymeric strand material includes a hydrophilic polyurethane. Other materials that are usable for either coatings for the aneurysm filler materials or the filler materials themselves include acrylamides such as hydroxypropyl methacrylamide, which is a hydrogel; isopropyl acrylamide, a thermoresponsive material; ethyl acrylamide, pH responsive material; and dicarboxymethylaminopropyl methacrylamide, which is a hydrogel. Other filler materials include methacrylates such as dimethyl amino ethyl methacrylate; oligo-dimethacrylate n-butyl acrylate, shape memory plastic; and hydroxyethyl methacrylate, which is a hydrogel. Other filler materials include cyclodextrans, synthetic elastin polymers such as protein gels, poly chelating and amphiphilic polymers.

Some embodiments of the one or more strands have one or more emboli dissolving agents released locally to reduce the emboli. In other embodiments, the strand releases oxygen and/or sugars to nourish the patient's brain cells. In other embodiments, the strand, or sleeve releases vasodilators such as nitrous oxide or heparin to increase the available oxygen transport. In other embodiments, the strand, or sleeve releases growth factors that improve healing or create new vessels.

For some embodiments, the one or more biocompatible polymeric strands are coated with one or more of collagen, fibrin, or other bioactive materials, that have been described herein, for rapid healing. It is understood that other materials, such as those described above, that aid in rapid healing are suitable for use in a coating. The coating is applied for some embodiments by deposition, and for other embodiments by application. In some examples, the biocompatible polymeric strands are made entirely of collagen.

A use of biodegradable materials provokes a wound healing response and concomitantly eliminates a mass effect of the filled aneurysm over time. For some embodiments, the biodegradable materials are seeded with materials such as growth factors, fibronectin, heparin, derivations of fibronectin, peptide mimics of fibronectin, laminin, vitronectin, thrombospondin, gelatin, collagen and subtypes thereof, gelatin, polylysine, polyornithine, and other adhesive molecules or derivatives or mimics of other adhesive molecules, integrins, cell attachment proteins, cells, and genes and gene products to speed cell overgrowth.

In various embodiments, the strand material described herein is one or more of polymeric and polymeric hybrids such as PEBAX, Grilamids, polyester, and silica. Materials also include reabsorbable materials such as PEG, poly(lactic-co-glycolic acid) ("PLGA"), and PLGA and base polymer. Materials further include textiles such as rayon, nylon, silk, Kyeon, Kevlar, and cotton. Materials also include biopolymers such as collagen, filaments, and coated polymeric material. Materials further include elastomers such as urethanes, silicones, nitriles, Teco Flux, carbothane, and silicone hybrids.

For some embodiments, lubricious materials such as hydrophilic materials are used to coat the multiple biocompatible polymeric strands. One or more bioactive materials are selected for a first biocompatible polymeric strand, and one or more materials other than the first materials are selected for a second biocompatible polymeric strand. If a guidewire or pushwire is used, the materials for that device are equivalent to biocompatible polymeric strand material, or are different.

Wire may also be any of a wide variety of stainless steels if some sacrifice of radiopacity is tolerated. Suitable materials of construction, from a mechanical point of view, are materials that maintain their shape despite being subjected to high stress. Certain "super-elastic alloys" include nickel/titanium alloys, copper/zinc alloys, or nickel/aluminum alloys.

Titanium/nickel alloys known as "nitinol" may also be used in wire embodiments. These are super-elastic and very sturdy alloys that will tolerate significant flexing without deformation even when used as a very small diameter wire. If nitinol is used in the wire, the diameter of the wire is significantly smaller than that of a core member that uses the relatively more ductile platinum or platinum/tungsten alloy as the material of construction.

The wire may also be made, in some embodiment, of radiolucent fibers or polymers (or metallic threads coated with radiolucent or radiopaque fibers) such as Dacron (polyester), polyglycolic acid, polylactic acid, fluoropolymers (polytetrafluoroethylene), Nylon (polyamide), or silk.

The term "bioactive" refers to any agent that exhibits effects *in vivo*, for example, a thrombotic agent, a therapeutic agent, and the like. Examples of bioactive materials include cytokines; extra-cellular matrix molecules (e.g., collagen); trace metals (e.g., copper); matrix metalloproteinase inhibitors; and other molecules that stabilize thrombus formation or inhibit clot lysis (e.g., proteins or functional fragments of proteins, including but not limited to Factor XIII, α2-antiplasmin, plasminogen activator inhibitor-1 (PAI- 1) or the like)). Examples of cytokines that are used alone or in combination in practicing the methods and apparatuses described herein include basic fibroblast growth factor (bFGF), platelet derived growth factor (pDGF), vascular endothelial growth factor (VEGF), transforming growth factor beta (TGF-β), and the like. Cytokines, extra-cellular matrix molecules, and thrombus stabilizing molecules are commercially available from several vendors such as Genzyme (Framingham, Mass.), Genentech (South San Francisco, Calif.), Amgen (Thousand Oaks, Calif.), R&D Systems, and Immunex (Seattle, Wash.). Additionally, bioactive polypeptides are synthesized recombinantly as the sequence of many of these molecules are also available, for example, from the GenBank database. Thus, it is intended that embodiments of the methods and apparatuses include use of DNA or RNA encoding any of the bioactive molecules.

Furthermore, molecules having similar biological activity as wild-type or purified cytokines, matrix metalloproteinase inhibitors, extra-cellular matrix molecules, thrombus-stabilizing proteins such as recombinantly produced or mutants thereof, and nucleic acid encoding these molecules are also used. The amount and concentration of the bioactive materials that are included in the composition of the core member vary, depending on the specific application, and are determined by one skilled in the art. It will be understood that any combination of materials, concentration, or dosage are used so long as it is not harmful to the subject.

In some embodiments, the distal portion 16 of the lumen includes a marker band. In various examples, the marker band is radioopaque. Materials for the marker band include, but are not limited to, barium sulfate, tantalum, gold, tungsten or platinum, bismuth oxide, bismuth subcarbonate, and the like. The use of the marker enables a physician to determine proper placement and proper fill in the aneurysm 24, in some examples.

The biocompatible polymeric strand or strands 26, in some embodiments, include a radioopaque marker. Materials for the marker include, but are not limited to, barium sulfate, tantalum, gold, tungsten or platinum, bismuth oxide, bismuth subcarbonate, and the like. The use of the marker enables a physician to determine proper placement and proper fill in the aneurysm 24. The markers are part of a single biocompatible polymeric strand or are part of two or more biocompatible polymeric strands of a weave. In some examples, the markers are bonded to loop around a biocompatible polymeric strand at a specified point along the length of the biocompatible polymeric strand, for example defining a ring around the biocompatible polymeric strand. Other marker configurations are possible.

FIG. 2 illustrates a biocompatible polymeric weave embolization device 200 including biocompatible polymeric strands joined at an end, according to an embodiment of the present subject matter. The illustration shows a twist design. The biocompatible polymeric strands 202 are shown abutting one another, but in some embodiments the biocompatible strands do not abut. In some examples, the biocompatible polymeric strands 202 are molded into the shown twist shape. Additionally, some examples plastically deform the biocompatible polymeric strands 202 while twisting. For example, it is possible to over-twist the biocompatible polymeric strands 202 and rely on a hysteresis effect on the biocompatible polymeric strand materials to result in the shown twist. In some examples, individual biocompatible polymeric strands are twisted or otherwise deformed prior to being twisted together. For example, biocompatible polymeric strands 202 are formed with a mechanical bias such that when they are paired they twist together to at least partially relieve the bias.

Biocompatible polymeric strands are joined at end portion or joint 204, in some embodiments. This joint 204 includes a bend in the biocompatible polymeric strands 202 in some instances. Additional examples include a joint 204 which includes a material which is not a continuous extension of one or more of the biocompatible polymeric strands, such as an adhesive, a weld including filler, or a combination thereof. Welds that do not include filler are also contemplated. Welding processes used include, but are not limited to, ultrasonic welds and laser welds.

The shown joint 204 is joined to the biocompatible polymeric strands such that the biocompatible polymeric strands 202 exhibit improved pushability. For example, when threading biocompatible polymeric strands 202 through a lumen and into an aneurysm, the joint 204 ensures that one or more of the biocompatible polymeric strands 202 do not get snagged. Various shapes are possible for the joint 204 such as the plate shape shown, as well as sphere shapes, semispherical shapes, bullet shapes, and other shapes.

FIG. 3 illustrates an embodiment of a biocompatible polymeric weave embolization device 300 including biocompatible polymeric strands joined together and joined at an end. The example shown is in an expanded state. For example, if the biocompatible polymeric weave embodiment of FIG. 3 is compressed axially along its length, and the biocompatible polymeric strands 302, 304 of the multiple biocompatible polymeric strands are not bonded to one another continuously along the length of the weave, they can expand from one another, such as by radial expansion of each biocompatible polymeric strand away from one another or away from a center axis of a weave. Such expansion is due to a mechanical bias, in some examples. Expansion is due to axial compression of the weave in additional examples.

Such expansion aids in filling the aneurysm. In examples which expand in use, such as those described herein, a higher degree of filling of an aneurysm is possible. Additionally, examples providing for such expansion offer increased flexibility during a filling procedure. In some examples, a lumen of a catheter is used to fix multiple biocompatible polymeric strands into an abutting state so that those biocompatible polymeric strands can be pushed through a catheter. Once the biocompatible polymeric strands are no longer located within a lumen, they are free to expand. Such freely expandable biocompatible polymeric strands are more easily folded into an aneurysm, such as when they are pushed into the wall of an aneurysm.

Bonded biocompatible polymeric strands are shown in FIG. 3. Such bonds are used in some examples to fix biocompatible polymeric strands to one another. Bond 306 is created while the biocompatible polymeric strands are in an expanded state. As such, these embodiments impart a mechanical bias which encourages the biocompatible polymeric strands 302, 304 to assume an expanded configuration. In some examples, bonds are created which impart a mechanical bias to keep biocompatible polymeric strands in a continuously abutting state, such as is shown in FIG. 3. Some examples use a range of bonds to impart varying mechanical biases. For example, a first bond encourages a first and second biocompatible polymeric strand to abut continuously along a first length of the string-shaped device. In the example, a second bond encourages the first and second biocompatible polymeric strands to not abut along a second length of the device. The bond 306 is formed in one of several ways, including adhesive bonding, welding, and combinations thereof.

Also illustrated in FIG. 3 illustrates a joint 308. The joint 308 can link two or more biocompatible polymeric strands according to various joint types, including, but not limited to, a molded joint, a weld, adhesive, etc. Bonds contemplated include adhesive bonds, weld bonds, and others. The shape of the joint 308 as shown is not limiting, as other shapes are possible.

FIG. 4 illustrates an embodiment of a biocompatible polymeric weave embolization device 400, according to an embodiment. Various filler configurations are possible, including, but not limited to, the shown weave. Also possible are a single strand, a single strand which is hollow, a composite strand including layers of different materials, a shaped fabric, or another structure discussed herein expressly. In some embodiments, one or more biocompatible polymeric strands are of a monolithic material and are free of lumens. A weave having biocompatible polymeric strands of different diameters, or which are solid, composite solid, or hollow are possible.

In some embodiments, the weave is string shaped including a length and an average width. An average width is measured by measuring different widths radially at a location on a main body 402, such as by pinching the biocompatible polymeric strands 408. An average width can also be measured by averaging a plurality of width measurements along the length of the main body 402, such as by pinching the biocompatible polymeric strands 408 at different locations along the length of the main body 402.

The example shown in FIG. 4 includes a main body 402 that includes multiple biocompatible polymeric strands 408 that are woven together to make the main body 402. Four biocompatible polymeric strands are shown in FIG. 4. Examples having two, three, and more than four are possible. Some examples include a pusher 406 and a weld 404 that attaches the biocompatible polymeric strands 408 to the pusher 406. The biocompatible polymeric strands 408 include any of the biocompatible polymeric strands discussed herein. In some examples, one or more of the biocompatible polymeric strands include a radioopaque material for radiographic use.

In some examples, the biocompatible polymeric strands are welded at periodic junctions to hold the biocompatible polymeric strands together. In some embodiments, the biocompatible polymeric strands are a product of a melt separation process, such as where a portion of the biocompatible polymeric strands are encapsulated in a low melting temperature material that is later melted away revealing the main body 402. For example, in a first portion of a catheter, the main body 402 is encapsulated in a material having a lower melting temperature than the biocompatible polymeric strands 408. In the example, in a second portion of the catheter, the encapsulation material is melted away.

In some examples, biocompatible polymeric strand embodiments are coated with a slippery coating. Some embodiments are coated with a bioactive coating. The biocompatible polymeric strands demonstrate varying durometer measurements when measured at different locations along the axis of the main body 402, in some examples. Biocompatible polymeric strands are of different durometers in some embodiments. For some embodiments, a single biocompatible polymeric strand has different durometers. For some embodiments, a distal section of selective biocompatible polymeric strands is removed to make the distal portion of the main body 402 more flexible. In some examples, a first biocompatible polymeric strand and a second biocompatible polymeric strand are in a weave and each exhibit different melting temperatures. In some examples, the first biocompatible polymeric strand is heated to its melting temperature by an external heat source, such as a heater coupled to a catheter, while a second biocompatible polymeric strand is not heated to its melting temperature.

In some examples, the main body 402 has a mechanical bias. Such biases are formed in a variety of ways. A bias is formed by building twist into a biocompatible polymeric strand before bonding the biocompatible polymeric strand to another biocompatible polymeric strand, so that the biocompatible polymeric strand tends to twist to a resting state other than the main body state 402. A bias is also formed by straightening a nonlinear biocompatible polymeric strand prior to bonding the biocompatible polymeric strand so that the biocompatible polymeric strand tends to bend. Some examples provide multiple biocompatible polymeric strands 408 with at least two of the biocompatible polymeric strands being weaved into the weave including biocompatible polymeric strands at different tensions such as to impart a mechanical bias, for example an arching main body 402.

In some embodiments, a weave includes a first biocompatible polymeric strand and a second biocompatible polymeric strand coupled together with at least a first joint and a second joint, with the first biocompatible polymeric strand having a mechanical bias away from the second biocompatible polymeric strand along a portion of the first biocompatible polymeric strand located between the first joint and the second joint. In some embodiments, the first joint includes a weld. In some embodiments, the mechanical bias includes a twist of the first biocompatible polymeric strand between the first joint and the second joint. In some embodiments, the mechanical bias is based on the first biocompatible polymeric strand having a molded shape other than one to position the first biocompatible polymeric strand adjacent the second biocompatible polymeric strand along the first portion.

In some examples, one of the biocompatible polymeric strands 408 is a guidewire. Some guidewires, and associated materials constituting a guidewire, are discussed herein, such as in the portion of the specification provided for FIG. 19. The main body 402 is weaved around a guidewire in some examples, such as where the main body 402 include flexible polymer, and the guidewire includes a less flexible material, such as a metallic material. In some embodiments a joint connecting strands is also joined to a guide wire. Joints which are joined to a guidewire provide a receptacle for a guidewire, such as by providing a socket shape for a guidewire to be inserted into and to push against. Other joint embodiments are possible.

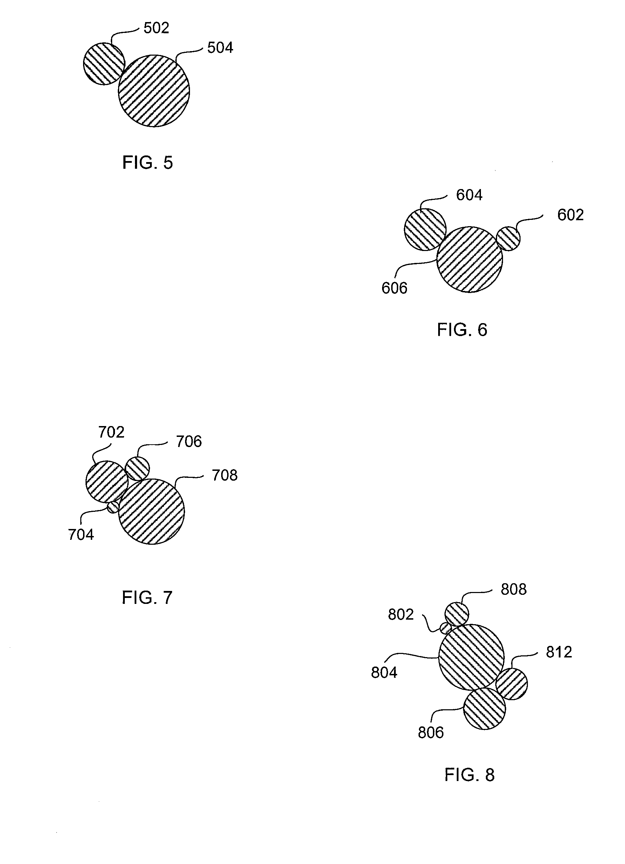

FIGS. 5-8 show different cross-section views of biocompatible polymeric strand assemblies, according to some examples. Biocompatible polymeric strands which have a generally circular cross section are shown. Biocompatible polymeric strands of differing sizes are shown to illustrate optional configurations which are used to provide one or more functions. One function is a time-delay dissolving of one or more biocompatible polymeric strands in sequence with other biocompatible polymeric strands. As such, a first biocompatible polymeric strand dissolves, allowing another to assume a bias or to become more flexible, such as in examples where two biocompatible polymeric strands are of differing stiffness.

FIG. 5 shows two biocompatible polymeric strands, with a first biocompatible polymeric strand 502 have a diameter which is smaller than that of a second biocompatible polymeric strand 504.

FIG. 6 shows three biocompatible polymeric strands, with a first 602 being the smallest, the second 606 being larger than the first, and with the third 604 optionally being larger than the second 606. Two of the biocompatible polymeric strands have the same diameters in some embodiments.

FIG. 7 shows four biocompatible polymeric strands 702, 704, 706, 708, which with an incrementally larger diameter than the previous.

FIG. 8 shows 5 biocompatible polymeric strands, 802, 804, 806, 808 and 812. These biocompatible polymeric strands have incrementally increasing cross section diameters. These cross sections are circular, but the present subject matter is not so limited. Two or more biocompatible polymeric strands have similar diameters. Two or more of the biocompatible polymeric strands in FIGS. 5-8 comprise different materials. Some of the biocompatible polymeric strands include a mechanical bias, in some embodiments.

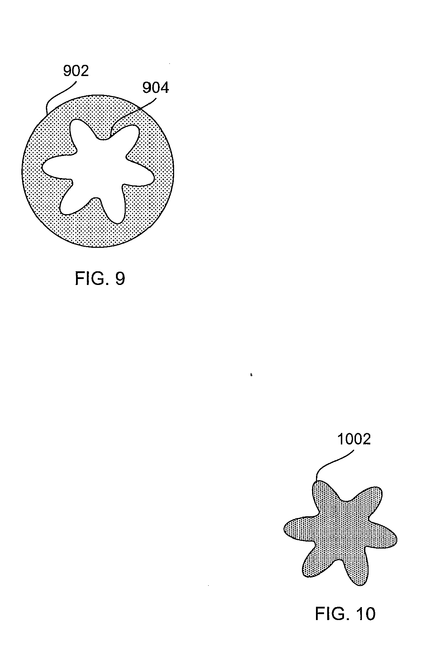

FIG. 9, illustrates a solid core 904 having a "star-like" shape and a fabric 902 adhered to the solid core 904 to form an annulus. FIG. 10, illustrates a "star-shaped" filler 1002. The filler 1002 in the embodiment is solid. In various embodiments, the textile materials are knits or woven and are expandable. The woven fabric or textile material includes, for some embodiments, one or more of polyester, nylon, absorbable material and fabric such as silk, suture material, and filter fabric. The textiles include polybutester such as Novatyil, PGA (Dexon), PLA (polylactic acid), polyglactin acid (Vicryl), polydiaxanone (POS) and polyglyconate (Maxon).

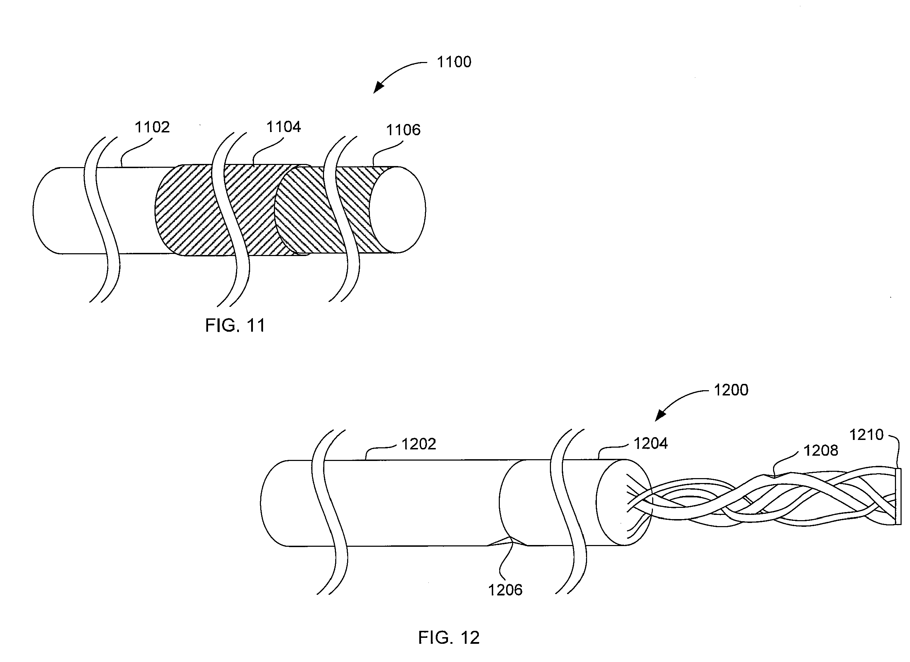

FIG. 11 illustrates a biocompatible polymeric pusher for pushing through a catheter, according to an embodiment. One biocompatible polymeric strand embodiment, shown at 1100 in FIG. 11 includes a solid biocompatible polymeric strand 1102, a pusher 1106 and an insert joint 1104 coupling the solid biocompatible polymeric strand 1102 and the pusher 1106. Although a single biocompatible polymeric strand is illustrated, a weave of biocompatible polymeric strands is possible.

The solid biocompatible polymeric strand(s) 1102 includes a solid strand that includes material that is inserted into the pusher 1106 at the insert joint 1104. The insert joint is part of the pusher in some examples. In additional examples, including the example show, the insert joint 1104 includes a material that can be collar shaped, which is interference fit over the pusher 1104. The pusher 1106 has high column strength imparted by, in some embodiments, PEEK. In various embodiments, the pusher includes one or more of Grilamids, nylon (12 30% glass (PARG)), polyamide, filled HDPE, polybutylene terephthalate, rigid polyurethane and polypropylene, which is 30% glass filled. For some embodiments, the insert joint 1104 attaches to the biocompatible polymeric strand and the pusher in an interference fit.

One benefit solid biocompatible polymeric strand 1102 is that it has a greater loading capacity than tungsten or a similar material is used without greatly impacting the integrity of the material. The solid biocompatible polymeric strand 1102 also has greater strength during delivery.

To detach the biocompatible polymeric strand, saline or contrast is used to pressurize the inside of the pusher and "inject" the biocompatible polymeric strand into the aneurysm. The pusher 1106 includes a distal portion that is used to push a biocompatible polymeric strand left behind in a microcatheter. Biocompatible polymeric strands or weaves may also be shipped in different lengths to accommodate different sized aneurysms. For some embodiments, the sleeve or strand is made of one or more polymers with a flex modulus within a range of 5 ksi to 200 ksi (kilopounds per square inch).

FIG. 12 illustrates a weave and a pusher, according to an embodiment. The embodiment includes a system 1200 that includes a pusher 1202 and a first end portion 1204, and a second end portion 1210. In some embodiments, a joint is disposed between the pusher 1202 and the first end portion 1204, to provide for detachability. In the shown embodiment, the first end portion 1204 is adhered to the pusher 1202. A first stress riser 1206 is provided. A stress riser is useful to encourage bending, in some embodiments. Additionally, such a stress riser encourages separation of the first end portion 1204 from the pusher 1202. Some embodiments include one or more stress risers 1208 on a weave, to encourage bending of the weave. The second end portion 1210 includes a weld, a knot, or another termination configuration. In some examples the weave is bonded to an end portion having a length and a diameter, the length of the pusher being inline with the length of the weave, the diameter of the cylindrical pushable biocompatible polymeric strand being approximately greater than the average width of the weave.

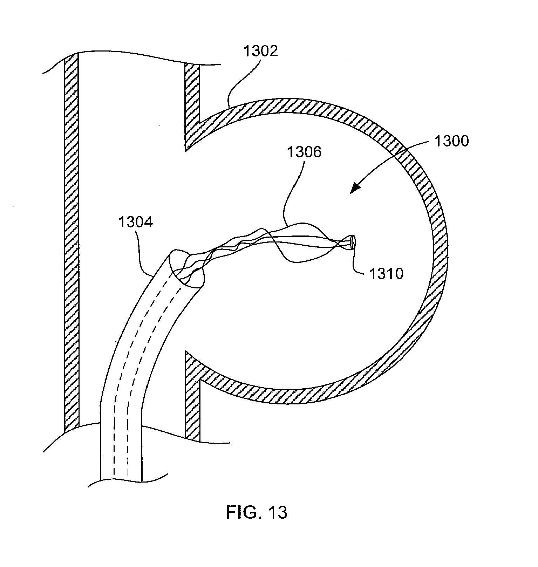

FIG. 13 illustrates an expanded weave embolization device in use, according to an embodiment. Illustrated is a weave 1300 that includes an end portion 1310 and a catheter 1304. The weave 1300 is to fill the aneurysm 1302. The weave expands in an expanded portion 1306 to better fill the aneurysm. In some embodiments, the weave is imparted with a bias to cause such expansion. As such, the weave expands as it exits the catheter 1304.

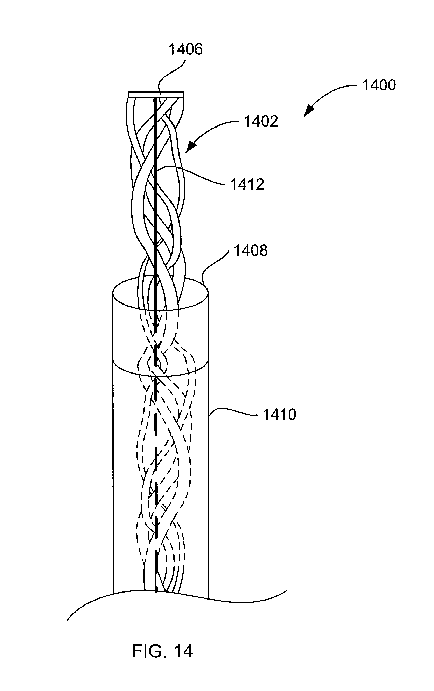

FIG. 14 illustrates an aneurysm filler system 1400 including a heated excise collar with a biocompatible polymeric strand slidably disposed in the catheter, according to an embodiment. Illustrated is a weave 1402 that includes an end portion 1406. In the illustration, the weave 1402 is pushed by a guidewire 1412. The weave extends through a catheter 1410 and a collar 1408 to an end portion 1406.

The collar 1408 can include a variety of sizes and shapes, as measured in diameters and lengths. In some examples, the collar 1408 is heated with direct DC power or other suitable energy sources. The power requirements of the detacher device are variable and depend upon the tissue requirements and the size of the device.

The biocompatible polymeric strand or weave is detachable at the catheter tip. Some embodiments do not require the catheter tip to enter the aneurysm, although the tip may enter the aneurysm. In some embodiments, the guidewire 1412 gains access and also functions as a rail to guide a biocompatible polymeric strand or strands into the aneurysm. The guidewire 1412 imparts strength and support sufficient to permit the biocompatible polymeric strand or weave to be pushed into the aneurysm without the material itself being reinforced.

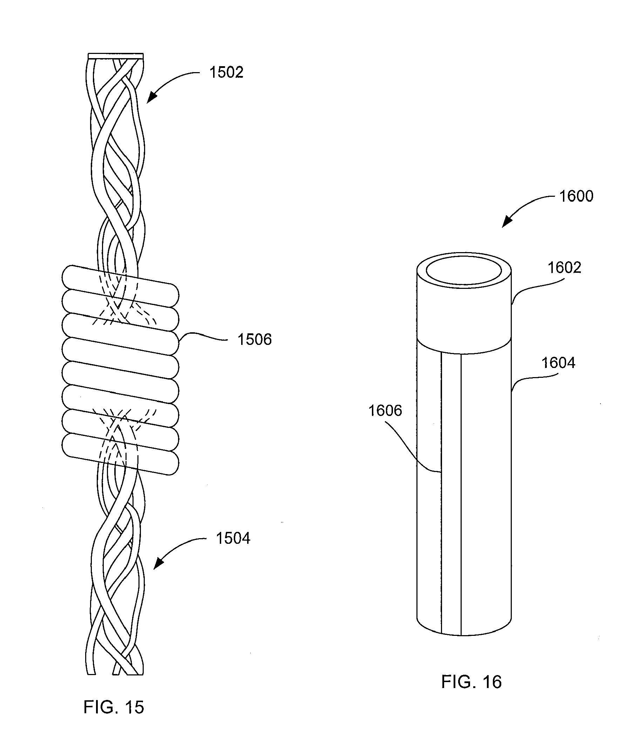

FIG. 15 illustrates a weave system including a conductive ring, according to an embodiment. The system includes a first weave portion 1502 and a second weave portion 1504 joined at a conductive ring 1506. The conductive ring 1506 is wrapped around a single weave, or it joins two biocompatible polymeric strand sets which have been weaved prior to coupling with the conductive ring 1506. The conductive ring 1506 is coil shaped, band shaped, wire shaped, or has another shape to circumscribe a biocompatible polymeric strand or weave. In various examples, a plurality of conductive rings is provided in a filler system.

The present subj ect matter provides filler via a catheter system 1600 which includes a conductive collar 1602, as illustrated in FIG. 16. A physician inserts a suitable amount of filler through a catheter 1604 and into an aneurysm and then excises the filler using at least one conductive ring 1506 paired to a conductive collar 1602. For example, a conductive ring 1506 is positioned inside a conductive collar 1602 such that the conductive ring 1506 is in electrical communication with the conductive collar 1602. The conductive collar 1602 is then electrified to heat the conductive ring to melt the filler. The present subject matter disposes unused conductive rings in an aneurysm. If conductive rings are positioned axially along filler at intervals, such as regular intervals, a surgeon monitors the amount of filler that has been disposed in the aneurysm. Although a single conductor 1606 is shown to provide power to the conductive collar 1602, embodiments having multiple conductors or leads are possible. Additionally, some embodiments include one or more electrically conductive biocompatible polymeric strands in a filler to energize the conductive collar 1602.

In some embodiments, the biocompatible polymeric strand or weave 1502 includes one or more electrically conductive leads embedded into the annular wall of the biocompatible polymeric strand or weave that connects to the conductive collar 1602, either at an inner annulus or an outer annulus, forming a circuit. In some embodiments, the biocompatible polymeric strand or weave includes a deposit of a flexible conductive film that contacts the collar 1602. In one other embodiment, a conductive layer is on an inner annular surface or an outer annular surface or both. Some embodiments enable a heated conductive collar 1602 to effectively detach a biocompatible polymeric strand or weave without the use of the conductor 1606.

Conductive strips and rings usable in the mechanism are made of one or more materials that include gold, platinum, silver, titanium, or tantalum. The conductive materials are corrosion resistant and coatings remain intact during the placement of the filler material. In some embodiments, the conductive materials are covered with a coating such as a hydrophilic coating to insulate the living being from the conductive materials. For some embodiments, growth factors such as those described herein overlay the conductive layer.

The conductive collar 1602 at a proximal portion of the aneurysm biocompatible polymeric strand or weave connects the conductive strips 1606 or electrical wires to lead wires going to a power supply. In another embodiment, the lead wires connect the conductive strips 1606 to a heat shrink/strain relief. In a third embodiment, the cylinder itself is conductive along its entire length. In some embodiments, a deposition coating forms a conductive ring on the outer diameter or inner diameter of the biocompatible polymeric weave. In another embodiment, an embedded conductive ribbon is inserted in the wall of the aneurysm biocompatible polymeric weave. The ribbon should be thin enough to break upon detachment. Thus, when the biocompatible polymeric strand or weave is excised, the circuit is broken. Such a break could activate an alarm system, in some examples.

Some embodiments of the detachment mechanism also includes a feedback mechanism for detachment that provides information to a physician as to when the strand or strands are excised. In addition to providing information concerning whether the strand or strands have been excised, the feedback mechanism may also provide information regarding the amount of filler added to the aneurysm, based upon the resistance measured prior to filler detachment. When current is applied, the resistance is measured. When the circuit is broken by excising the aneurysm filler, the resistance is measured again. The change in resistance is associated with the length of the aneurysm filler added to the aneurysm.

In some embodiments, at the proximal portion, a conductive cylinder is connected to an electrical circuit monitoring device that senses the resistance of the conductive cylinder. With this embodiment, a calculation is performed to determine the amount of filler added to an aneurysm. The resistance is proportional to the amount of filler added. The wires or flexible conductive coating extend from a hub to a distal portion of the aneurysm filler biocompatible polymeric strand or weave in a way effective for creating an electrical circuit. The filler along with elements that conduct an electric current create a closed electrical circuit. When the filler is excised, the circuit is opened.

In one other embodiment, when the aneurysm filler biocompatible polymeric strand or weave is excised with heat, the electrical circuit is also excised, thus sending a change in resistance or change in the circuit integrity to the circuit-monitoring device which triggers an alarm indicating the change in the circuit. In this fashion, a user of the device will have a positive feedback of the separation of the material without having to visualize the separation or tactilely feel the separation occur.

Various embodiments of the present subject matter combine the devices illustrated in FIGS. 15-16 to operate in various ways. When filler is inserted into the aneurysm, the catheter excises the filler so that all of the coils in the aneurysm will not be activated. A second way that component B functions is to run multiple copper lengths up to a weave and then electrify the collar 1602 using energy traveling over the copper length. A third method is to have a set length of material and place the coil at a length in the material so that the same amount of material is excised every time.

For some embodiments, conductor(s) 2208 are embedded or adhered to a wall of sheath connected to a conductor layer and bound at a distal portion which connects to a coil to close the electrical circuit for activation of a heater coil.

For some embodiments, the system includes a conductive layer or band that is not a continuous band. Rather, the band is of discrete length and both bands are offset to allow current to flow through the heater coil. For some embodiments, the conductive collar 1602 could be used optionally to cauterize or ablate tissue. The cauterization or ablation is performed *in situ*.

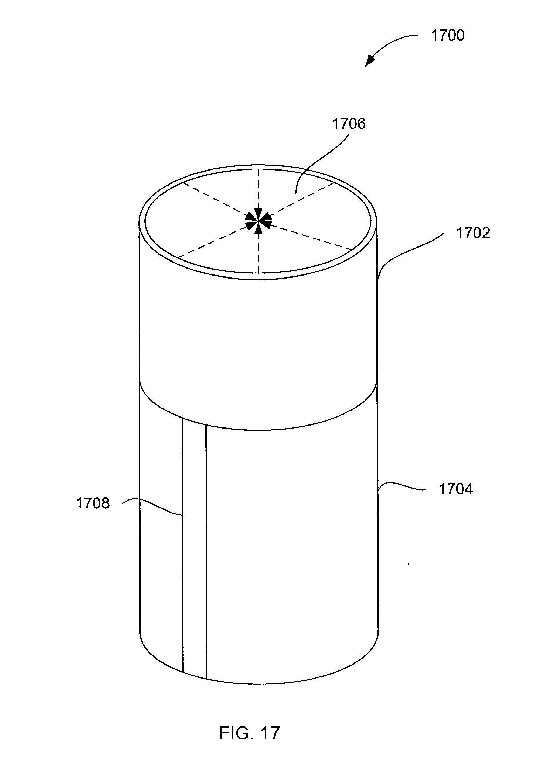

FIG. 17 illustrates a catheter system 1700 having a heated excise collar demonstrating isotropic heating, according to an embodiment. Various embodiments provide a conductive collar 1702 coupled to the distal portion of the catheter 1704. In various embodiments, the heated excise collar provides radial heat 1706 from the catheter inward to melt and excise a biocompatible polymeric strand or weave. The excise collar is provided with energy along a conductor 1708. The lead carries thermal and/or electrical energy, in various embodiments. The conductive collar 1702 is a band, a coil, a wire or another shape to circumscribe a weave or biocompatible polymeric strand.

The collar 1702 is adapted to provide isotropic heat, in some embodiments, via coatings applied to the conductive collar. For example, the conductive collar, in some embodiments, is disposed inside a distal portion of a catheter which has a melting temperature which is greater than that of the biocompatible polymeric weave to be melted upon disposition through and activation of the conductive collar 1702. In some examples, the exterior of the catheter includes a thermally insulative coating enveloping the heated excise collar, such as paint or similar coating. In some embodiments, the conductive collar 1702 provides anisotropic heat. Anisotropic heat, in some embodiments, is provided by a composite such as an epoxy composite, including carbon nanotubes.

For some embodiments, the aneurysm filler includes a segment that includes a polymer having a melting point that is lower than the rest of the aneurysm filler. The segment is positioned to cover an area where the filler will be excised. The segment is large enough to increase flexibility of where the filler detachment occurs. In another embodiment, the segment includes materials that promote a sharp melting point.

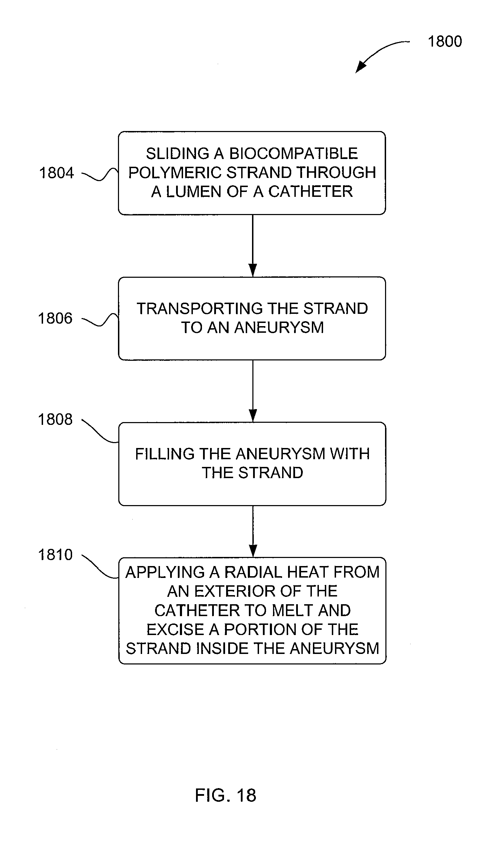

FIG. 18 illustrates a method of embolization, according to an embodiment. At 1804, the embodiment includes sliding a biocompatible polymeric strand through a lumen of a catheter. Multiple biocompatible polymeric strands are used. At 1806, the embodiment includes transporting the biocompatible polymeric strand to an aneurysm. At 1808 the embodiment includes filling the aneurysm with the biocompatible polymeric strand. At 1810 the embodiment includes applying a radial anisotropic heat from an exterior of the catheter inward to melt and excise a portion of the biocompatible polymeric strand inside the aneurysm.

Various optional methods are contemplated. Systems, methods and apparatus are included in which material of the biocompatible polymeric strand includes one or more of the materials described herein. Weaves including multiples strands of multiple respective materials are contemplated.

Various methods are contemplated including those which use anisotropic heat to melt and excise the biocompatible polymeric strand, the isotropic heat being less than the heat needed to melt the catheter. Some methods including weaving the biocompatible polymeric strand into a weave with at least one other biocompatible polymeric strand, the weave being string shaped. Some methods include bonding the weave to a pusher. Some embodiments include transporting the biocompatible polymeric strand to an aneurysm which further comprises pushing the weave through the catheter.

Referred to herein are trade names for materials including, but not limited to, polymers and optional components. The inventors herein do not intend to be limited by materials described and referenced by a certain trade name. Equivalent materials (e.g., those obtained from a different source under a different name or catalog (reference) number to those referenced by trade name may be substituted and utilized in the methods described and claimed herein. All percentages and ratios are calculated by weight unless otherwise indicated. All percentages are calculated based on the total composition unless otherwise indicated. All component or composition levels are in reference to the active level of that component or composition, and are exclusive of impurities including but not limited to residual solvents or by-products, which may be present in commercially available sources.

The application extends to a method for treating an aneurysm by at least partially filling it with a biocompatible polymeric strand, comprising: sliding the biocompatible polymeric strand through a lumen of a catheter; transporting the biocompatible polymeric strand to the aneurysm; filling the aneurysm with the biocompatible polymeric strand; and applying a radial heat from an exterior of the catheter inward to melt and excise a portion of the biocompatible polymeric strand inside the aneurysm. The method may further comprise weaving the biocompatible polymeric strand into a weave with at least one other biocompatible polymeric strand, the weave being string shaped. The method extend further to bonding the weave to a pusher. Transporting the biocompatible polymeric strand to an aneurysm may further comprise pushing the weave through the catheter. The material of the biocompatible polymeric strand comprises one or more of a group including an acrylamide, a methacrylate, cyclodextran, synthetic elastin polymer, poly chelating amphiphilic polymers, hydrogels, hyaluronic acid conjugates, polyanhydrides, glycolipids, polysaccharides, and halamines, natural hydrogel, a synthetic hydrogel, silicone, polyurethane, polysulfone, cellulose, polyethylene, polypropylene, polyamide, polyimide, polyester, polytetrafluoroethylene, polyvinyl chloride, epoxy, phenolic, neoprene, polyisoprene, and a combination thereof. Applying a radial heat from an exterior of the catheter inward to melt and excise a portion of the biocompatible polymeric strand inside the aneurysm may comprise applying a radial anisotropic heat. The method may further comprise using anisotropic heat to melt and excise the biocompatible polymeric strand, the isotropic heat being less than the heat needed to melt the catheter.

Although detailed embodiments of the invention are disclosed herein, it is to be understood that the disclosed embodiments are merely exemplary of the invention that may be embodied in various forms. Specific structural and functional details disclosed herein are not to be interpreted as limiting, but merely as a basis for teaching one skilled in the art to variously employ the aneurysm filler detacher wire embodiments. Various modifications may be made by those skilled in the art without departing from the spirit and scope of the invention.

## Claims

1. An apparatus, comprising:
a biocompatible polymeric weave including a plurality of biocompatible polymeric strands in a weave, with each of the biocompatible polymeric strands made from a material selected from one or more of a group consisting of an acrylamide, a methacrylate, synthetic elastin polymer, poly chelating amphiphilic polymers, hydrogels, hyaluronic acid conjugates, polyanhydrides, glycolipids, polysaccharides, and halamines, natural hydrogel, a synthetic hydrogel, silicone, polyurethane, polysulfone, cellulose, polyethylene, polypropylene, polyamide, polyimide, polyester, polytetrafluoroethylene, polyvinyl chloride, epoxy, phenolic, neoprene, polyisoprene, and a combination thereof,
wherein a first biocompatible polymeric strand and a second biocompatible polymeric strand are coupled together with at least a first joint and a second joint, with the first biocompatible polymeric strand having a mechanical bias away from the second biocompatible polymeric strand along a portion of the first biocompatible polymeric strand located between the first joint and the second joint.

2. The apparatus of claim 1, wherein the first joint includes a weld.

3. The apparatus of claim 1 or claim 2, wherein the mechanical bias includes a twist of the first biocompatible polymeric strand between the first joint and the second joint.

4. The apparatus of claim 1, wherein the mechanical bias is based on the first biocompatible polymeric strand having a molded shape other than one to position the first biocompatible polymeric strand adjacent the second biocompatible polymeric strand along the weave.

5. A system, comprising:
a catheter comprising a main body having a distal portion for location at least partially in an aneurysm and a proximal portion, the catheter to transport a biocompatible polymeric strand to the aneurysm;
at least one biocompatible polymeric strand sized to slide through the catheter; and
an excise collar coupled to the distal portion of the catheter, the excise collar active to provide radial heat from the catheter inward to melt and excise the biocompatible polymeric strand.

6. The system of claim 5, wherein the biocompatible polymeric strand is in a weave with a plurality of other biocompatible polymeric strands such that the weave is string shaped including a length and an average width.

7. The system of claim 6, further comprising a guidewire, with the weave woven about the guidewire.

8. The system of claim 6 or claim 7, comprising a plurality of welds disposed axially along the length of the weave between a first biocompatible polymeric strand and a second biocompatible polymeric strand.

9. The system of any one of claims 6 to 8, wherein the biocompatible polymeric strand comprises fabric.

10. The system of any one of claims 6 to 8, wherein the biocompatible polymeric strand is hollow.

11. The system of any one of claims 6 to 10, wherein the biocompatible polymeric strand is monolithic and is free of lumens.

12. The system of any one of claims 6 to 11, wherein the weave has a nonlinear mechanical bias.

13. The system of any one of claims 6 to 11, wherein the weave is bonded to an end portion of a pusher having a length and a diameter, the length of a pusher being inline with the length of the weave, the diameter of the pusher being approximately greater than the average width of the weave.

14. The system of claim 13, wherein the pusher comprises one or more of Grilamids, nylon (12 30% glass (PARG)), polyamide, filled HDPE, polybutylene terephthalate, rigid polyurethane and 30% glass filled polypropylene.

15. The system of any one of claims 5 to 14, wherein the biocompatible polymeric strand comprises material selected from one or more of an acrylamide, a methacrylate, synthetic elastin polymer, poly chelating amphiphilic polymer, hydrogel, hyaluronic acid conjugate, natural hydrogel, a synthetic hydrogel, silicone, polyurethane, polysulfone, cellulose, polyethylene, polypropylene, polyamide, polyimide, polyester, polytetrafluoroethylene, polyvinyl chloride, epoxy, phenolic, neoprene, polyisoprene, one or more of a thermoresponsive polymer, a pH sensitive polymer, or a shape memory polymer and a combination thereof.

16. The system of anyone of claims 5 to 15, wherein the biocompatible polymeric strand comprises a conductive ring effective to thermally conducting heat from the collar.

17. The system of anyone of claims 5 to 16, wherein an exterior of the catheter comprises a thermally insulative coating enveloping the excise collar.

18. The system of anyone of claims 5 to 17, wherein the excise collar is to provide anisotropic heat.
